# FASCICULE DE BREVET EUROPEEN

(11) **EP 2 855 534 B1**
(45) Date de publication et mention de la délivrance du brevet: **10.08.2016**
(21) Numéro de dépôt: 13730029.9
(22) Date de dépôt: 03.06.2013
(51) Int. Cl.: C08B 15/04, A61L 15/28, D06M 11/30, D06M 13/477

(54) **MATERIAU A BASE DE CELLULOSE OXYDEE, SON PROCEDE D'OBTENTION ET SON UTILISATION COMME COMPRESSE**
OXIDIERTES CELLULOSEBASIERTES MATERIAL, VERFAHREN ZUM ERHALT DAVON UND VERWENDUNG DAVON ALS KOMPRESSE
OXIDIZED CELLULOSE-BASED MATERIAL, METHOD FOR OBTAINING SAME AND USE THEREOF AS COMPRESS

(30) Priorité: 04.06.2012 FR 1255182
(43) Date de publication de la demande: 08.04.2015
(73) Titulaire: Symatese, 69630 Chaponost (FR)
(72) Inventeur: DAO, Vithuy, F-94260 Fresnes (FR); MICHELOT, Robert, F-92160 Antony (FR); HERBAGE, Benjamin, F-69350 La Mulatiere (FR); FUCHEZ, Fabien, F-69770 Montrottier (FR); PEROUSE, Eric, F-75016 Paris (FR)
(74) Mandataire: Cabinet Laurent & Charras
(86) Numéro de dépôt international: PCT/FR2013/051255
(87) Numéro de publication internationale: WO 2013/182798

(56) Documents cités:
- EP-A1- 0 492 990
- EP-A1- 1 215 217
- EP-A1- 1 462 123
- EP-A1- 2 279 762
- WO-A1-00/50462
- WO-A1-01/34656
- WO-A1-2012/119229
- US-A- 4 543 410

## Description

### DOMAINE DE L'INVENTION

La présente invention propose un nouveau matériau solide à base de polymère contenant des unités cellobiose au moins partiellement oxydées, susceptible d'être utilisé dans le domaine médical, notamment en tant que compresse.

Un tel matériau, avantageusement un textile, peut être obtenu en soumettant un matériau solide à base d'un polymère contenant des unités cellobiose à un procédé en au moins deux étapes : une mise en contact avec un mélange oxydant comprenant un hypohalite, un halite, et un sel d'oxoammonium ou un précurseur dudit sel ; la mise en contact du matériau ainsi traité avec une solution d'acide periodique ou de l'un de ses sels ; puis éventuellement la mise en contact du matériau ainsi traité avec un halite.

### ETAT DE LA TECHNIQUE

Les compresses hémostatiques chirurgicales, ou compresses, doivent être hémostatiques, résorbables et facilement manipulables par les chirurgiens. Ces propriétés peuvent être obtenues grâce à des textiles à base de cellulose oxydée.

Pour rappel, la cellulose est un homopolymère appartenant à la classe des polysaccharides. Elle est formée d'un enchainement linéaire de molécules de glucose ou unités anhydroglucose (D-anhydroglucopyranose), reliées entre elles par des liaisons glycosidiques β-1,4. La cellulose peut être aussi définie comme un enchainement d'unités cellobiose :

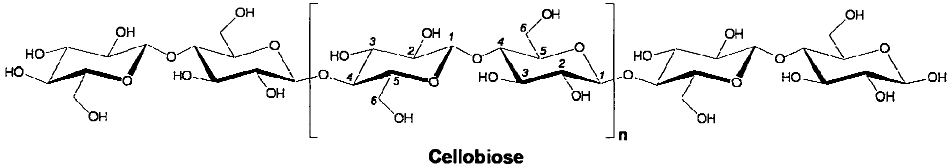

Actuellement, de telles compresses sont obtenues en soumettant le textile à base de cellulose à l'action du NO₂ en présence de solvants, tel que décrit par exemple dans le document EP 0 492 990. Ainsi, le produit de référence, actuellement commercialisé sous le nom de Surgicel^{®}, est obtenu à l'aide d'un tel procédé.

Toutefois, cette technologie requiert des matières premières coûteuses, dangereuses et difficiles à recycler, ainsi qu'une installation exigeante. C'est pourquoi, il a été tenté de développer d'autres voies pour obtenir des compresses.

Le document WO 2009/016325 décrit un procédé d'oxydation alternatif de la cellulose au sein d'un tissu, à l'aide d'un système catalytique oxydant comprenant du 1-oxo-2,2,6,6-tétraméthylpipéridine-1-oxyde (TEMPO), du NaBr et du NaClO. Cette réaction se déroule à basse température et à un pH basique devant être strictement contrôlé pendant la réaction.

Parallèlement, il est connu par exemple du document EP 2 216 345, que l'oxydation de la cellulose par un système oxydant comprenant du TEMPO, du NaClO et du NaClO₂ conduit à une cellulose partiellement oxydée sélectivement au niveau de l'alcool primaire en C₆, présentant un degré de polymérisation élevé, une bonne résistance mécanique et une bonne stabilité à la lumière.

Par ailleurs, Singh *et al.* ont décrit un procédé d'oxydation de la cellulose, basé sur l'utilisation du periodate de sodium. La cellulose ainsi traitée, qui présente des fonctions aldéhyde et une ouverture de cycle, montre une résorption *in vivo* lente. Ces fonctions aldéhyde peuvent être utilisées pour immobiliser notamment des enzymes d'intérêt.

Il existe toutefois un besoin évident de développer de nouvelles compresses de cellulose oxydée. Des propriétés recherchées pour de telles compresses sont notamment :
- des propriétés hémostatiques satisfaisantes ;
- une résorption contrôlable, avantageusement de l'ordre de 2 semaines après implantation dans l'organisme ;
- une bonne stabilité mécanique ;
- susceptibles d'être obtenues à l'aide d'un procédé peu coûteux et facilement transposable au niveau industriel.

### EXPOSE DE L'INVENTION

La présente invention propose un nouveau matériau solide à base de cellulose oxydée, utilisable en tant que compresse et susceptible d'être obtenu grâce à un procédé original.

Ainsi et selon un premier aspect, la présente invention propose un matériau solide à base d'un polymère contenant des unités cellobiose, dans lequel :
- au moins une partie des unités cellobiose présente au moins une des deux fonctions alcool primaire, portée par le carbone C₆, oxydée en fonction acide carboxylique ; et
- au moins une partie des unités cellobiose présente au moins un des deux cycles, ouvert entre les carbones C₂ et C₃.

Dans le cadre de la présente invention, on entend par « matériau solide » aussi bien des fibres ou micro fibrilles, que des assemblages de fibres ou des fibres solidarisées, sous la forme d'un fil, ou sous la forme d'un tissu formé de fils, tissés ou tricotés par exemple, ou bien sous la forme d'un non tissé, ou bien sous la forme d'une nappe de fibres formant une matrice orientée, ou bien encore sous forme de mousse ou de poudre.

Dans le cadre de la présente invention, on comprend par « fil », un assemblage linéaire de fibres (ou de microfilaments) liées entre elles de manière solidaire. Un fil de cellulose est donc à distinguer d'une fibre de cellulose, qui désigne un objet individuel et non un assemblage solidaire d'objets. Selon un mode de réalisation particulier, le matériau solide ne correspond pas à des fibres individualisées. Typiquement, un fil est obtenu par filage de fibres du même type, mais peut également être obtenu par filage de fibres différentes, comme par exemple des fibres de cellulose en combinaison avec des fibres synthétiques.

Dans un mode de réalisation privilégié, les fils sont mis sous forme de tissu à l'aide du tricotage. Différentes mailles peuvent être envisagées, notamment le jersey (classiquement tricoté avec un fil de 220 dtex à 42 filaments) ou le crochet (avantageusement tricoté avec du fil 110 dtex à 40 filaments).

De manière privilégiée, le matériau solide est un textile. Dans le cadre de l'invention, on entend par « textile » un assemblage de fils ou de fibres, avantageusement solidarisées entre eux ou entre elles, formant une entité solide et insoluble. Ainsi et de manière appropriée, le textile peut être un tissu, avantageusement obtenu par tissage ou tricotage de fils, ou un non tissé obtenu par assemblage de fibres.

Le polymère constitutif des fibres du matériau selon l'invention contient des unités cellobiose. Il peut par exemple s'agir de cellulose naturelle ou de cellulose modifiée. Dans le cas de la cellulose modifiée, il s'agit avantageusement de la viscose, qui correspond à de la cellulose régénérée.

Les fibres mises en oeuvre selon l'invention sont obtenues par exemple à partir de cellulose naturelle, c'est-à-dire de fibres directement issues d'un végétal, soit en étant récoltées sur le végétal, soit en étant obtenues par un traitement mécanique du végétal, comme par un broyage, un pressage, un concassage et/ou une séparation. Les fibres de cellulose sont également des fibres de cellulose modifiée, c'est-à-dire de cellulose naturelle ou de cellulose naturelle solubilisée, ayant réagi avec un composant chimique.

Le terme « fibres de cellulose » inclut aussi, au sens de la présente invention, les fibres de cellulose régénérées, c'est-à-dire les fibres de cellulose naturelle, éventuellement modifiée, solubilisées dans un solvant, puis reformées sous forme de fibres.

Des exemples de fibres de cellulose naturelle d'origine végétale sont le coton, le chanvre, le jute ou la pulpe de bois. Ces fibres de cellulose peuvent aussi être d'origine bactérienne.

Les fibres de cellulose artificielle sont obtenues par un procédé de traitement de la cellulose naturelle.

L'expression « à base d'un polymère contenant des unités cellobiose » signifie que ce polymère constitue l'ingrédient majeur voir unique du matériau. Toutefois, il n'est pas exclu que le matériau comprenne d'autres constituants, par exemple au moins un autre polymère ne contenant pas d'unités cellobiose tel que des polymères constitutifs des fibres synthétiques. Dans un mode de réalisation particulier, d'autres polymères contenant éventuellement des unités anhydroglucose peuvent être associés, tels que l'alginate, l'acide hyaluronique, l'amidon ou d'autres glycosaminoglycanes.

On entend par « polymère contenant des unités cellobiose » un polymère qui comprend au moins deux unités cellobiose dans sa chaîne et dans un cas extrême qui est constitué uniquement d'unités cellobiose, tel que la cellulose.

Selon l'invention, ledit polymère est modifié, au moins partiellement, au niveau de ses unités cellobiose.

Selon une première caractéristique privilégiée du polymère, au moins une partie des unités cellobiose présente au moins une des deux fonctions alcool primaire, portée par le carbone C₆, oxydée en fonction acide carboxylique. En d'autres termes, la fonction portée par le carboné C₆ est soit une fonction alcool soit une fonction acide carboxylique. La présence d'une fonction aldéhyde au niveau du C₆ est donc avantageusement exclue.

Au niveau d'une unité cellobiose du polymère, plusieurs cas de figure peuvent se présenter :
- les deux unités anhydroglucose formant l'unité cellobiose présentent une fonction alcool primaire (non modifiée) en C₆ ;
- les deux unités anhydroglucose formant l'unité cellobiose présentent une fonction acide carboxylique en C₆ ;
- une des deux unités anhydroglucose formant l'unité cellobiose présente une fonction acide carboxylique en C₆, l'autre présentant une fonction alcool en C₆.

Selon une seconde caractéristique privilégiée du polymère, au moins une partie des unités cellobiose présente au moins un des deux cycles ouvert entre les carbones C₂ et C₃. En d'autres termes, les carbones C₂ et C₃ peuvent être non modifiés, c'est-à-dire qu'ils forment un cycle et portent une fonction alcool. Ainsi, la présence d'une fonction cétone au niveau de C₂ et/ou C₃ est avantageusement exclue. Alternativement, le cycle entre les carbones C₂ et C₃ peut être ouvert. Dans ce cas de figure et de manière avantageuse, les carbones C₂ et/ou C₃ peuvent porter des fonctions aldéhyde et/ou acide carboxylique, éventuellement fonctionnalisées. Des fonctions acétal et semi-acétal, résultant de la réaction des groupements alcool avec les groupes aldéhyde, peuvent également être présentes.

A nouveau, au niveau d'une unité cellobiose du polymère, plusieurs cas de figure peuvent se présenter :
- les deux unités anhydroglucose présentent des carbones C₂ et C₃ engagés dans le cycle et porteurs d'une fonction alcool (non modifiés) ;
- les deux unités anhydroglucose présentent un cycle ouvert entre les carbones C₂ et C₃, ceux-ci portant avantageusement des fonctions aldéhyde et/ou acide carboxylique, éventuellement fonctionnalisées ;
- une des deux unités anhydroglucose formant l'unité cellobiose présente un cycle ouvert entre les carbones C₂ et C₃, ceux-ci portant avantageusement des fonctions aldéhyde et/ou acide carboxylique, éventuellement fonctionnalisées ; l'autre unité anhydroglucose est non modifiée, c'est-à-dire que les carbones C₂ et C₃ sont engagés dans le cycle et portent une fonction alcool.

Selon l'invention, ces deux caractéristiques doivent se retrouver dans le polymère, au niveau d'au moins une unité cellobiose ou éventuellement au niveau d'au moins deux unités cellobiose distinctes, chacune portant une des deux caractéristiques décrites ci-dessus.

Dans le cas où le polymère contenant des unités cellobiose est de la cellulose ou de la viscose, le polymère constitutif du matériau selon l'invention est donc un dérivé de cellulose ou de viscose, plus précisément un dérivé oxydé.

Selon un mode de réalisation privilégié et comme il sera détaillé ci-dessous, la première caractéristique peut être conférée au matériau en le soumettant à l'action d'un mélange oxydant comprenant un hypohalite, un halite et un sel d'oxoammonium ou un précurseur dudit sel. Toutefois, toute méthode de conversion du groupement alcool primaire porté par le carbone C₆ exclusivement en groupement acide carboxylique constitue une alternative envisageable à l'utilisation du mélange oxydant décrit ci-dessus. Ainsi d'autres procédés permettant l'oxydation sélective du carbone en C₆ d'une unité anhydroglucose peuvent être mis en oeuvre, notamment à l'aide d'ions nitrosonium (essentiellement TEMPO et dérivés), avec ou sans catalyse par des éléments de transition, avec ou sans NaBr, avec ou sans enzyme oxydante, avec ou sans agent complexant, tel que par exemple décrit dans US 6,716,976.

Ainsi et à ce stade, au moins une partie, éventuellement toutes les unités anhydroglucose du polymère sont converties en unités glucuronique. Le polymère en présence correspond donc à un acide polyglucuronique.

Sans vouloir être lié à une quelconque théorie, cette première étape permettrait de contrôler l'acidité du tissu, propriété importante pour l'hémostase. Toutefois, à l'issue de cette étape, le matériau obtenu est peu résorbable, propriété améliorée grâce à la mise en oeuvre de la deuxième étape du procédé selon l'invention :
Selon un mode de réalisation privilégié et comme il sera décrit ci-dessous, l'ouverture du cycle entre les carbones C₂ et C₃ est obtenue en soumettant le matériau à l'action d'une solution d'acide periodique ou de l'un de ses sels. Là encore, tout procédé alternatif permettant l'ouverture du cycle entre C₂ et C₃ et l'oxydation des fonctions alcool portées par ces carbones peut être mis en oeuvre.

De manière connue, le traitement par le periodate entraîne, outre l'ouverture du cycle entre les carbones C₂ et C₃, l'oxydation des fonctions alcool portées par ces carbones en fonctions aldéhyde. Ces fonctions peuvent servir au greffage de molécules d'intérêt, telles que des enzymes, permettant ainsi de fonctionnaliser le matériau.

En outre, ces fonctions aldéhyde peuvent être converties en fonctions acide carboxylique. Ces fonctions acide carboxylique peuvent également être fonctionnalisées, de manière à générer par exemple des fonctions ester ou amide.

Ainsi, le matériau selon l'invention est riche en fonctions acide carboxylique qui peuvent être portées par :
- le carbone en C₆ ;
- le carbone en C₆ et le carbone en C₂ ;
- le carbone en C₆ et le carbone en C₃ ;
- le carbone en C₆, le carbone en C₂ et le carbone en C₃.

A noter que dans le cadre de l'invention, les fonctions acide carboxylique peuvent être sous forme protonée ou sous forme d'ion carboxylate, par exemple complexé à du calcium.

De manière avantageuse, le degré ou taux d'oxydation global du matériau selon l'invention, qui correspond à la conversion des fonctions alcool et/ou aldéhyde en fonctions acide carboxylique, est supérieur à 10%, avantageusement supérieur à 12%, voire supérieur à 15%.

Pour rappel, le degré ou taux d'oxydation est défini comme la masse des groupements acide carboxylique contenus dans 100g du matériau. La mesure de l'oxydation du polymère, en particulier de la cellulose, peut être évaluée par exemple par titrimétrie, selon le protocole décrit par Sobue et Okubo en utilisant la méthodologie décrite dans la Pharmacopée US, par résonnance magnétique nucléaire (RMN) (Kumar *et al*.), ou encore par spectrométrie infra rouge (Fujisawa *et al*.).

Pour rappel et selon l'invention, le polymère doit présenter les deux caractéristiques précitées. En pratique, la chaîne polymérique peut présenter :
- des unités cellobiose présentant uniquement une ou les deux fonctions alcool primaire, portées par le carbone C₆, oxydées en fonction acide carboxylique. L'éventuel carbone C₆ ne portant pas de fonction acide carboxylique porte une fonction alcool primaire. Par ailleurs, ces unités cellobiose restent non modifiées au niveau des carbones C₂ et C₃, c'est-à-dire que ceux-ci sont engagés dans le cycle et portent une fonction alcool ;

- des unités cellobiose présentant uniquement un ou les deux cycles ouverts entre les carbones C₂ et C₃. L'éventuel cycle non ouvert entre C₂ et C₃ porte des fonctions alcool au niveau des carbones C₂ et C₃. Par ailleurs, ces unités cellobiose restent non modifiées au niveau du carbone C₆, c'est-à-dire que celui-ci porte une fonction alcool primaire ;
- des unités cellobiose présentant à la fois au moins une des deux fonctions alcool primaire portée par le carbone C₆, voire les deux, oxydée en fonction acide carboxylique et au moins un des deux cycles ouvert entre les carbones C₂ et C₃, voire les deux, comme illustré dans le schéma ci-dessous pour une unité anhydroglucose de cellobiose dans laquelle les carbones C₂ et C₃ portent des fonctions aldéhydes :

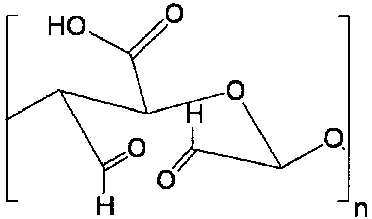

Dans le cadre de la présente invention, il a été démontré qu'un matériau solide, notamment sous forme de textile et présentant les caractéristiques structurales énoncées ci-dessus, pouvait être utilisé en tant que compresse médicale, susceptible de présenter les propriétés attendues en terme de stabilité mécanique, de tenue au vieillissement, de propriétés hémostatiques et de caractère résorbable.

Dans le cadre de l'invention, on entend par « stabilité mécanique » le fait que le matériau soit manipulable avec des ustensiles, notamment des pinces, ou même manuellement, tout en conservant une bonne tenue mécanique. La stabilité mécanique confère également au matériau la propriété de pouvoir être conservé plusieurs mois, voire plusieurs années, à température ambiante, sans que son intégrité soit altérée.

Dans le cadre de l'invention, on entend par « hémostatique » le fait que le matériau soit capable d'arrêter les saignements lorsqu'il est appliqué localement.

Dans le cadre de l'invention, on entend par « caractère résorbable » le fait que le matériau soit totalement ou majoritairement dégradé *in vivo* 2 semaines après implantation, avantageusement après 1 semaine. La résorption peut être quantifiée visuellement, en observant le degré de dégradation du matériau, ou par tout autre méthode adaptée comme l'histologie par exemple.

En fonction de sa structure, le matériau selon l'invention présente un caractère résorbable modulable. Ainsi, selon les applications envisagées, il peut être implanté pour des durées variables : une implantation à court terme, se traduisant par une résorption totale du matériau au terme d'une durée maximale de 30 jours ou une implantation à long terme se traduisant par une résorption complète du matériau au terme d'une durée supérieure à 30 jours.

De manière générale, la présente invention présente un intérêt dans le domaine médical, ledit matériau pouvant être utilisé en tant que compresse, pansement implantable, prothèse, endoprothèse vasculaire ou matrice de recellularisation, pour une implantation ou une régénération d'organe.

Avantageusement, il s'agit d'une compresse. De manière classique, une compresse peut se présenter sous forme d'éponge ou de textile. Dans le cadre de l'invention, une compresse correspond préférentiellement à un textile obtenu à l'aide du procédé revendiqué.

Plus généralement, l'utilisation première du matériau solide selon l'invention dépend du type de matériau en présence :
- les fibres ou micro fibrilles peuvent être associées dans un non-tissé ou une mousse, d'où l'utilisation du matériau selon l'invention pour l'élaboration d'un non-tissé ou d'une mousse ;
- les fibres ou micro fibrilles peuvent être associées dans un fil, d'où l'utilisation du matériau selon l'invention pour l'élaboration d'un fil ;
- un fil peut être tissé ou tricoté pour former un tissu, d'où l'utilisation du matériau selon l'invention pour l'élaboration d'un tissu ;
- un tissu peut être utilisé comme compresse, d'où l'utilisation du matériau selon l'invention en tant que compresse ;
- les fibres ou micro fibrilles peuvent être divisées pour obtenir une poudre.

Dans le cadre de l'invention, il a été mis en évidence un procédé d'obtention d'un matériau présentant l'ensemble des caractéristiques mentionnées ci-dessus. Plus précisément, l'invention vise un procédé permettant d'obtenir un matériau solide à base d'un polymère dont les unités cellobiose présentent les caractéristiques suivantes :
◆ au moins une partie des unités cellobiose présente au moins une fonction acide carboxylique porté par le carbone C₆, les autres carbones C₆ portant une fonction alcool primaire ; et
◆ au moins une partie des unités cellobiose présente au moins un des deux cycles ouvert entre les carbones C₂ et C₃, les autres carbones C₂ et C₃ formant un cycle et portant une fonction alcool.

Selon l'invention, ce procédé comprend les étapes suivantes :
- une première étape de mise en contact d'un matériau solide à base de polymère contenant des unités cellobiose et d'un mélange oxydant comprenant un hypohalite, un halite et un sel d'oxoammonium ou un précurseur dudit sel ; puis
- une deuxième étape de mise en contact du matériau ainsi traité et d'une solution d'acide periodique ou de l'un de ses sels.

Selon un autre aspect, la présente invention vise un procédé de traitement d'un matériau solide à base de polymère contenant des unités cellobiose comprenant les étapes suivantes :
- une première étape de mise en contact du matériau solide et d'un mélange oxydant comprenant un hypohalite, un halite et un sel d'oxoammonium ou un précurseur dudit sel ; puis
- une deuxième étape de mise en contact du matériau ainsi traité et d'une solution d'acide periodique ou de l'un de ses sels.

Selon un mode de réalisation particulier et dans une troisième étape, le matériau ainsi traité est mis en contact avec une solution d'halite.

Ce procédé peut être mis en oeuvre soit directement sur le matériau déjà mis en forme, à savoir un textile, soit sur des fils qui seront ensuite tissés ou tricotés, voire sur des fibres qui seront ensuite associées par électrospinning ou cardage/aiguilletage sous forme de non tissé, orienté ou non, ou de fils. De manière avantageuse, le procédé selon l'invention est mis en oeuvre sur un matériau solide présentant sa forme finale, avantageusement sous forme de textile.

Il est toutefois envisageable de changer de forme de matériau entre les étapes. Ainsi et à titre d'exemple, la première étape peut être mise en oeuvre sur des fibres qui pourront, entre les 2 étapes, être assemblées sous forme de non tissé ou de fil. De même, la première étape peut être réalisée sur des fils qui seront ensuite tricotés ou tissés, la deuxième étape étant réalisée sur le tricot ou le tissu.

Avantageusement en amont du procédé selon l'invention, le matériau peut être soumis à une étape de désensimage permettant l'élimination des corps gras utilisés dans la filature. Ces corps gras sont essentiellement constitués d'huiles d'ensimage et de colle acrylate enduits autour des filaments. Différents protocoles de désensimage sont connus de l'art antérieur et peuvent être mis en oeuvre.

Selon un mode de réalisation particulier, le matériau solide à base de polymère comprenant des unités cellobiose, soumis au procédé selon l'invention, est dépourvu de cellulose oxydée.

Par « dépourvu de cellulose oxydée », on entend, au sens de la présente invention, présentant un degré d'oxydation inférieur à 1%. Il s'agit de préférence des fibres de cellulose naturelle ou synthétique, ou bien des fibres synthétiques ou bien des fibres naturelles contenant éventuellement de la cellulose, mais dépourvues de cellulose oxydée, c'est-à-dire n'ayant pas reçu de traitement chimique oxydant particulier.

Par fibres synthétiques, on entend des fibres qui n'ont aucun polymère précurseur naturel, tel que les fibres obtenues par polymérisation d'un monomère synthétique, par exemple dérivé du pétrole.

Les fibres synthétiques, résorbables ou non, comprennent notamment les polyamides tels que les nylons, les polyesters, les polyacrylates, les polyuréthannes, des acides polylactiques et polyglycoliques, les polyéthylènes glycols, les poly-α-oléfines et les polymères halogénés, leur copolymères ou leur combinaison.

La première étape du procédé selon l'invention est donc celle permettant l'oxydation sélective d'au moins un groupement alcool primaire situé en position 6 des unités cellobiose présentes dans le matériau solide.

Cette première étape est réalisée en présence d'un hypohalite, d'un halite et d'un sel d'oxoammonium ou d'un précurseur dudit sel, avantageusement en solution aqueuse. Dans le cadre de la présente invention, on entend par « précurseur de sel d'oxoammonium » une espèce chimique propre à générer un sel d'oxoammonium par réaction avec un des constituants du mélange oxydant, notamment par oxydation par un des constituants du mélange.

Les sels d'oxoammonium sont des oxydants hydrosolubles capables d'oxyder sélectivement les alcools primaires, lorsque la réaction se déroule dans des conditions appropriées de pH et de température. Les sels d'oxoammonium privilégiés selon l'invention sont les sels di-tert-alkylés, en particulier les sels de 1-oxo-2,2,6,6-tétraméthylpipéridine-1-oxyde, appelés communément TEMPO, et ses dérivés.

Les sels d'oxoammonium, catalyseurs de cette première étape d'oxydation partielle, sont régénérés *in situ* par un système oxydant secondaire ou auxiliaire, en l'occurrence en présence d'un hypohalite, préférentiellement l'hypochlorite de sodium (NaClO). Ce dernier est à son tour régénéré *in situ* par la réaction d'un halite, préférentiellement le chlorite de sodium NaClO₂ après qu'il ait réagi avec les aldéhydes nouvellement formés par oxydation de l'alcool primaire en position 6.

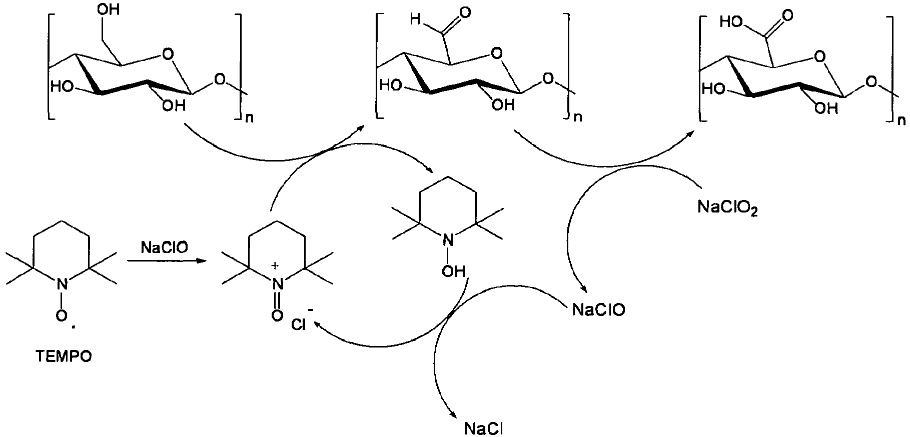

Selon un mode de réalisation préféré de l'invention, la première étape d'oxydation est réalisée en présence d'une quantité substoechiométrique de sel d'oxoammonium ou de l'un de ses précurseurs par rapport à la quantité d'unités anhydroglucose présentes dans le matériau traité. En pratique et de manière adaptée, le sel d'oxoammonium ou l'un de ses précurseurs représente de 0,0003 à 0,0006 mole par gramme de polymère contenant des unités cellobiose (cellulose).

A noter que pour les quantités de réactifs mentionnées dans la description, celles-ci correspondent au cas particulier où le polymère contenant des unités cellobiose est de la cellulose. Ainsi, dans le cas d'un mélange de polymères ou de polymères ne présentant pas uniquement des unités cellobiose, ces quantités sont calculées en fonction du nombre d'unités cellobiose présentes.

Selon un autre mode de réalisation préféré de l'invention, la première étape d'oxydation est réalisée en présence d'une quantité en hypohalite substoechiométrique par rapport à la quantité d'unités anhydroglucose présentes dans le matériau traité, avantageusement de 0,0006 à 0,0049 mole d'hypohalite par gramme de polymère contenant des unités cellobiose (cellulose). Typiquement, une quantité de 0,0012 mole d'hypohalite par gramme de polymère contenant des unités cellobiose (cellulose) est utilisée.

Selon un autre mode de réalisation préféré de l'invention, la première étape d'oxydation est réalisée en présence d'un excès stoechiométrique en halite par rapport à la quantité d'unités anhydroglucose présentes dans le matériau traité, avantageusement de 0,006 à 0,025 mole d'halite par gramme de polymère contenant des unités cellobiose (cellulose). Typiquement, une quantité de 0,012 mole d'halite par gramme de polymère contenant des unités cellobiose (cellulose) est utilisée.

Selon un mode de réalisation particulier, ces 3 constituants sont présents dans le mélange oxydant dès le début de la réaction.

De manière avantageuse, le mélange oxydant est formulé dans de l'eau déminéralisée, de manière à ne pas perturber la force ionique et le pH.

Selon un autre aspect de l'invention, la première étape d'oxydation est avantageusement réalisée dans un milieu réactionnel dont le pH est neutre, voire légèrement acide, avantageusement compris entre 5 et 7. En d'autres termes, cette première réaction est réalisée à pH compris entre 5 et 7.

Selon un autre aspect de l'invention, cette étape d'oxydation est préférentiellement réalisée à une température supérieure à 40°C, avantageusement de l'ordre de 60°C.

Typiquement, cette réaction d'oxydation se déroule pendant une durée comprise entre 1 et 6 heures, avantageusement de 4 à 5 heures.

Selon un mode de réalisation particulier, le matériau obtenu à l'issue de cette première étape présente un taux d'oxydation compris entre 10 et 16%, avantageusement compris entre 12 et 15%.

Un moyen radical pour stopper la réaction d'oxydation est d'ajouter au milieu réactionnel un alcool primaire, tel que l'éthanol, en excès qui va réagir avec le sel d'oxoammonium et diluer considérablement par effet de masse la réaction avec la cellulose.

A l'issue de cette première étape, il peut être utile de protoner les fonctions acide carboxylique présentes dans le matériau ainsi traité.

De manière classique, cette étape est réalisée par incubation du matériau dans un milieu de protonation. Ceci est avantageusement réalisé par incubation dans de l'acide chlorhydrique (HCl), avantageusement dans un ou plusieurs bains de HCl de 0,1 à 1 mol/l (N), pendant plusieurs heures.

En pratique et pour éviter des manipulations du matériau traité, le milieu d'oxydation peut être éliminé par vidange et remplacé par le milieu de protonation.

A l'issue de cette première étape d'oxydation, et éventuellement de protonation, le matériau est avantageusement lavé et séché.

Le lavage de la compresse se fait de manière avantageuse dans un milieu contenant un solvant et/ou de l'eau. Un alcool et/ou de l'eau déminéralisée et/ou de l'acétone constituent un milieu privilégié de lavage, par exemple de l'éthanol à 50% puis de l'éthanol à 95%.

Ces lavages peuvent être répétés et durent généralement quelques heures, avantageusement de 1 à 10 heures.

De manière appropriée, le matériau ainsi traité est séché par tout moyen adapté, notamment à l'air en statique ou dynamique ou encore sous vide, et ce avantageusement pendant plusieurs heures.

La deuxième étape d'oxydation est réalisée en présence d'acide periodique, ou l'un de ses sels, dans le milieu réactionnel. En d'autres termes, il s'agit d'incuber le matériau dans une solution d'acide periodique, ou de l'un de ses sels. Il s'agit avantageusement d'une solution aqueuse.

L'ion periodate est connu pour couper les glycols et les transformer en dialdéhydes. De cette façon, il n'y a pas coupure de la chaine polymérique, mais plutôt acquisition d'une plus grande flexibilité par ouverture des cycles. Dans le cadre de l'invention, il a été montré qu'il était possible de moduler les propriétés de résorption du matériau traité en jouant sur l'intensité (temps, température, concentration des réactifs, ...) de cette étape : plus la réaction est « poussée », plus le matériau obtenu se résorbe rapidement.

De manière avantageuse, l'acide periodique ou l'un de ses sels est présent à hauteur de 0,003 à 0,012 mole par gramme de polymère contenant des unités cellobiose (cellulose), avantageusement 0,006 mole par gramme de polymère contenant des unités cellobiose (cellulose).

Avantageusement, le sel d'acide periodique est le periodate de sodium.

Selon un autre aspect de l'invention, la deuxième étape d'oxydation est réalisée à un pH compris entre 2 et 5, avantageusement égal à 3.

Selon un autre aspect de l'invention, cette deuxième étape est réalisée à une température comprise entre 5 et 60°C, avantageusement égale à 35°C.

En outre, la durée classique de cette étape est avantageusement comprise entre 1 et 6 heures, avantageusement égale à 3h.

De manière avantageuse, cette réaction se déroule à l'abri de la lumière.

Comme déjà dit, une troisième étape d'oxydation peut être mise en oeuvre sur le matériau solide cellulosique ayant subi les deux premières étapes d'oxydation.

Cette troisième étape est alors réalisée en présence d'un halite, avantageusement en présence d'une solution aqueuse d'halite. Cette étape permet l'oxydation sélective des fonctions aldéhydes obtenues après la réaction au periodate en fonctions acide carboxylique. De préférence, l'halite est un chlorite, et encore plus préférentiellement le chlorite de sodium.

De manière avantageuse, la solution comprend de 0,0025 à 0,012 mole d'halite par gramme de polymère contenant des unités cellobiose (cellulose), avantageusement 0,006 mole par gramme de polymère contenant des unités cellobiose (cellulose).

Selon un mode préféré de réalisation, la troisième étape est réalisée à un pH compris entre 5 et 7, avantageusement égal à 5,8.

La température à laquelle se déroule cette réaction est avantageusement supérieure à 15°C, encore plus avantageusement égale à 35°C. Alternativement, elle peut être réalisée à température ambiante.

Typiquement, la troisième réaction d'oxydation du matériau cellulosique est réalisée pendant une durée comprise entre 0,25 et 2 heures, avantageusement de 0,5 à 1 heure.

La conversion des fonctions aldéhyde en fonctions acide carboxylique permet, grâce à la mesure du taux d'oxydation, de quantifier la réaction ayant lieu à la seconde étape du procédé selon l'invention. Ainsi et selon un mode de réalisation privilégiée, la combinaison des étapes 2 et 3 permet une augmentation du taux d'oxydation comprise entre 1 et 7%, avantageusement entre 2 et 4%.

Ainsi et selon la présente invention, l'oxydation des unités cellobiose a lieu selon le schéma suivant :

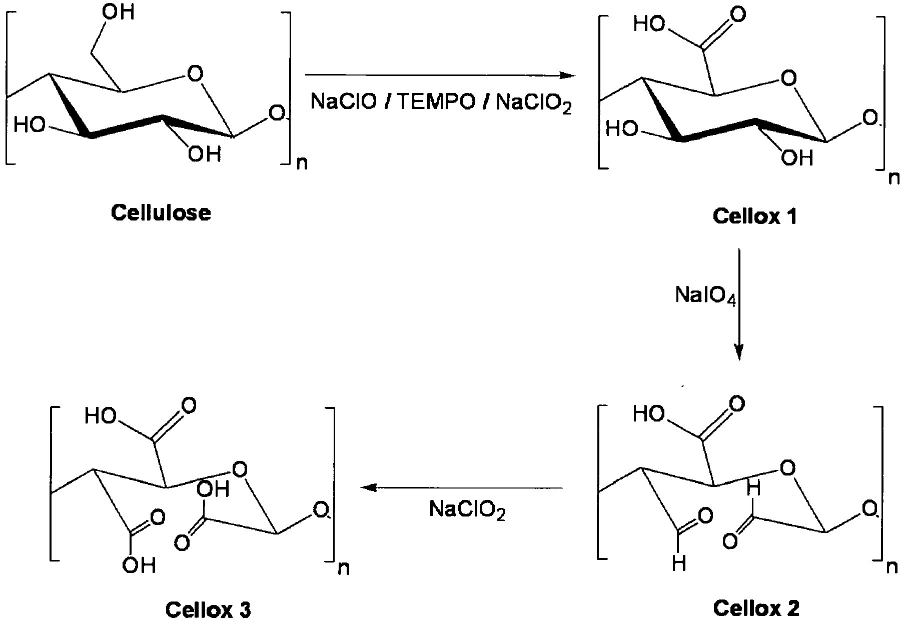

De manière avantageuse, à l'issue de chacune des étapes du procédé selon l'invention, le matériau est lavé dans un bain d'eau distillée et/ou d'éthanol et/ou d'acétone, éventuellement de manière répétée. Il est ensuite avantageusement séché comme décrit ci-dessus.

Ensuite, une éventuelle étape d'acidification ou de protonation, comme décrite ci-dessus, peut être mise en oeuvre.

D'autre part et de manière connue, un matériau à base de cellulose oxydée doit présenter soit des fonctions acide carboxylique protonées, soit complexées avec des ions calcium (Ca²⁺), pour développer les propriétés hémostatiques recherchées.

Dans le cas où il est souhaité un matériau oxydé présentant des fonctions acide carboxylique complexées avec des ions calcium (Ca²⁺), plusieurs possibilités existent :
- soit l'oxydation est mise en oeuvre à l'aide de réactifs se présentant sous forme de sels de calcium. Dans ce cas, à l'issue de la réaction, les fonctions acide carboxylique se présentent sous la forme (COO)₂Ca.
- alternativement, il peut être envisagé de réaliser l'incubation en présence de la source de calcium (notamment acétate de calcium ou CaCl₂), après l'oxydation ;
- une troisième possibilité consiste à incuber la compresse dans un milieu contenant une source de calcium, après l'étape de protonation. Là encore, des sources de calcium particulièrement adaptées sont l'acétate de calcium ou le CaCl₂. Dans cette nouvelle étape, les ions Ca²⁺ vont remplacer les protons des fonctions carboxylates.

A l'issue du procédé selon l'invention, le matériau traité subit un ou plusieurs lavages, avantageusement à l'éthanol, et un séchage, réalisés dans les conditions décrites ci-dessus.

Il peut également subir des traitements liés à l'application visée : conditionnement en emballages individuels, stérilisation, etc.

A noter que, comme démontré dans le cadre de la présente demande, l'ordre des deux premières étapes du procédé est crucial, notamment lorsqu'il est mis en oeuvre sur un textile. En effet, l'inversion des deux étapes entraîne une dégradation du textile.

Le procédé selon l'invention susceptible d'être mis en oeuvre sur tout matériau solide, et notamment sur un textile, présente de nombreux avantages parmi lesquels :
- il met en oeuvre des réactifs peu coûteux, facilement manipulables et non dommageables pour l'environnement, contrairement au NO₂.
- Lorsqu'il est mis en oeuvre sur un textile, il permet d'obtenir un produit présentant des caractéristiques de résistance mécanique, d'hémostase et de résorption compatibles avec une utilisation en tant que compresse.
- Il permet de contrôler le degré et la régiosélectivité des réactions d'oxydation de manière précise. Ainsi, la première étape d'oxydation permet d'obtenir un degré d'oxydation supérieure ou égale à 10% voire supérieure ou égale à 12% ; la deuxième étape, lorsqu'elle est suivie d'une conversion des fonctions aldéhyde en acide carboxylique, permet encore d'augmenter le niveau d'oxydation au moins de 1%, voire de 7%.
- Il permet de contrôler les modifications oxydatives, alors que d'autres techniques telles que le NO₂ sont non sélectives : les fonctions alcool attaquées ainsi que les groupements formés ne sont pas contrôlés. A titre d'exemple, le NO₂ attaque le C₆ mais également les C₂ et C₃, au niveau desquels il engendre la formation aussi bien d'aldéhyde, d'acide carboxylique que de cétone (sans coupure du cycle dans ce dernier cas), tout en laissant des traces d'azote sur le produit final.

### EXEMPLES DE REALISATION

L'invention et les avantages qui en découlent ressortiront mieux des exemples de réalisation présentés ci-dessous qui n'ont toutefois aucune portée limitative.

Ces exemples sont basés sur des tissus fabriqués à partir de fils de cellulose régénérée (ou viscose) tricotés, en maille crochet ou jersey.

### I/ Procédé d'obtention du matériau solide cellulosique :

### 1) Première étape :

Cette étape permet l'oxydation partielle de la cellulose via la conversion de fonctions alcool primaire portées par le carbone C₆ des unités cellobiose en fonctions acide carboxylique.

Les conditions générales de cette étape sont décrites dans le Tableau 1 ci-dessous :

| | |
|---|---|
| TEMPO (mole par gramme de cellulose) | 0,0003 à 0,0006, par exemple 0,0006 |
| NaClO (mole par gramme de cellulose) | 0,0006 à 0,0049, par exemple 0,0012 |
| NaClO₂ (mole par gramme de cellulose) | 0,006 à 0,025, par exemple 0,012 |
| pH du milieu réactionnel | 5 à 7, par exemple 5,8 |
| Température (°C) | 60 |
| Durée de la réaction (h) | 1 à 6, typiquement de 4 à 5 |

Sous une hotte aspirante, 200 ml d'une solution tampon d'acétate de sodium (0,5 M, pH = 5,8) sont placés dans un erlen de 1 1, puis 0,12 mole de NaClO₂ sont ajoutés. 10 g de cellulose régénérée tricotée sont ensuite placés dans la solution de NaClO₂. 0,006 mole de TEMPO et une solution aqueuse de NaClO à 2% en chlore actif (0,012 mol) sont ensuite ajoutés au mélange réactionnel. Le volume total du mélange réactionnel est 500 ml. L'erlen est fermé et placé dans un bain-marie thermostaté maintenu à une température de 60°C pendant une durée de 1 à 6 h, et typiquement de 4 à 5 h. L'oxydation est arrêtée par addition d'un excès d'éthanol.

Le tissu de cellulose partiellement oxydée est essoré puis incubé pendant 12 h dans une solution aqueuse d'HCl 1N sur un agitateur orbital. La solution aqueuse d'HCl 1N est renouvelée et l'incubation est poursuivie pendant 2 h.

Le tissu de cellulose partiellement oxydée est essoré, puis lavé avec de l'éthanol à 50% pendant 1 h sur un agitateur orbital. Cette opération est renouvelée deux fois. Le tissu est alors essoré, puis lavé avec de l'éthanol à 95% pendant 1 h sur un agitateur orbital, puis enfin essoré et laissé sécher à l'air sous hotte aspirante pendant au moins 12 h.

### 2) Deuxième étape:

Cette étape permet l'ouverture du cycle entre les carbones C₂ et C₃ des unités cellobiose. Simultanément, les fonctions alcool portées par ces carbones sont oxydées en fonctions aldéhyde.

Les conditions générales de cette étape sont présentées dans le Tableau 2 ci-dessous :

| | |
|---|---|
| NaIO₄ (mole par gramme de cellulose) | 0,003 à 0,012, par exemple 0,006 |
| pH du milieu réactionnel | 2 à 5, par exemple 3 |
| Température (°C) | 5 à 60, par exemple 35 |
| Durée de la réaction (h) | 1 à 6, par exemple 3 |

10 g du matériau obtenu à l'issue de la première étape d'oxydation partielle sont mélangés avec 0,06 mole d'ion periodate dans un erlen de 1 1. Le volume total du mélange réactionnel est 500 ml. Le pH est ajusté à 3 et la réaction est réalisée sous agitation pendant 3 h, à une température de 35°C et à l'abri de la lumière. Le matériau cellulosique est ensuite rincé deux fois à l'eau distillée avant de subir une acidification et un lavage à l'éthanol, comme décrit plus haut.

### 3) Troisième étape (optionnelle) :

Cette étape permet l'oxydation des fonctions aldéhyde portées par les carbones C₂ et C₃ des unités cellobiose en fonctions acide carboxylique.

Les conditions générales de cette étape sont présentées dans le Tableau 3 ci-dessous :

| | |
|---|---|
| NaClO₂ (mole par gramme de cellulose) | 0,0025 à 0,012, par exemple 0,006 |
| pH du milieu réactionnel | 5 à 7, par exemple 5,8 |
| Température (°C) | Supérieure à 15°C, par exemple 35°C |
| Durée de la réaction (h) | 0,25 à 2, par exemple 1 |

10 g du matériau obtenu à l'issue des deux premières étapes sont mélangés avec 0,06 mole de chlorite de sodium préparé dans un tampon acétique à pH 5,8. La réaction est réalisée pendant 30 minutes, à la température ambiante. Le volume total du mélange réactionnel est d'environ 150 ml. Le matériau cellulosique est ensuite rincé deux fois à l'eau distillée avant de subir une acidification et un lavage à l'éthanol, comme décrit plus haut. Le matériau est ensuite séché.

### 4) Suivi de la formation des fonctions aldéhyde,

La présence de fonctions aldéhyde a été suivie grâce au réactif Purpald^{®} qui révèle leur présence par l'apparition d'une coloration pourpre intense.

L'intensité de cette coloration a été gradée pour différents produits: de 0 pour une solution qui ne présente pas de coloration pourpre à +++ pour une solution pourpre très intense.

Les résultats sont présentés dans le Tableau 4 ci-dessous :

| Produit testé | Textile de cellulose non traité | Textile de cellulose après la première étape | Textile de cellulose après la seconde étape | Surgicel® |
|---|---|---|---|---|
| Intensité de la coloration pourpre | 0 | 0 | +++ | ++ |

Ces expériences révèlent que la première étape ne génère pas de fonctions aldéhyde au niveau du C₆, alors que la deuxième étape entraîne concomitamment la coupure du cycle entre les carbones C₂ et C₃ et la génération de fonctions aldéhyde au niveau de ces carbones. Ce test s'avère également positif sur une compresse Surgicel^{®} obtenu par un traitement au NO₂.

### II/ Evaluation des propriétés physiques du matériau

### 1) Degré d'oxydation du tissu obtenu :

Le degré d'oxydation du tissu de cellulose partiellement oxydée représente la masse des groupements acide carboxylique contenus dans 100 g dudit tissu. Cette valeur est mesurée par titrimétrie, selon la méthode d'échange de calcium décrite par Sobue et Okubo, et recommandée par l'United States Pharmacopeia (USP, 1990).

Le degré d'oxydation de différents tissus (essai 1 : crochet ; essai 2 : jersey), obtenu à l'aide du procédé en deux étapes (TEMPO/NaClO/NaClO₂ puis NalO₄), a été mesuré.

Les conditions expérimentales et les degrés d'oxydation mesurés sont présentés dans le Tableau 5 ci-dessous :

| | | **Essai 1** | **Essai 2** |
|---|---|---|---|
| **Etape** | **Nature du tissu de cellulose** | crochet | Jersey |
| | **TEMPO (mol/g de cellulose)** | 0,0006 | 0,0006 |
| | **NaClO (mol/g de cellulose)** | 0,0012 | 0,0012 |
| **1** | **NaClO₂ (mol/g de cellulose)** | 0,012 | 0,012 |
| | **Température (°C)** | 60 | 60 |
| | **Durée de la réaction (h)** | 6 | 6 |
| | **NaIO₄ (mol/g de cellulose)** | 0,006 | 0,006 |
| **2** | **Température (°C)** | 35 | 35 |
| | **Durée de la réaction (h)** | 3 | 3 |
| **Degré d'oxydation produit (%)** | | 13,44 | 13,55 |

En conclusion, le taux d'oxydation est suffisant pour que le matériau obtenu à l'aide du procédé selon l'invention possède de bonnes propriétés hémostatiques.

### 2) Stabilité du tissu obtenu :

Le tissu de cellulose partiellement oxydée obtenu conserve une excellente résistance mécanique après 1 mois, dans une étuve à 60°C.

Cet échantillon conserve un bon aspect, sans dégradation. Ce même échantillon a été stérilisé par irradiation aux rayons β, puis a été soumis à un procédé de vieillissement qui n'a pas provoqué de dégradation du tissu.

Une stabilité équivalente à celle du produit Surgicel^{®} a également été observée après 3 mois à 25°C et en humidité contrôlée, ou après un stress thermique à 60°C.

### 3) Degré de polymérisation apparent du tissu obtenu :

La mesure du degré de polymérisation apparent a été réalisée par la mesure de la viscosité suivant la norme NF G06-037 (décembre 1981).

Les résultats obtenus sont présentés dans le Tableau 6 ci-dessous :

| **Produit** | **Degré de polymérisation** |
|---|---|
| **Cellulose non traitée** | 270 |
| **Surgicel® (produit de référence)** | 20 |
| **Crochet (Essai 1)** | 20 |
| **Jersey (Essai 2)** | 20 |

En conclusion, le procédé selon la présente invention aboutit à un matériau (essais 1 et 2) avec un degré de polymérisation apparent, mesuré par la viscosité, du même ordre que le produit de référence (Surgicel®).

### III/ Evaluation des propriétés précliniques du matériau

### 1) Résorption :

Les études précliniques ont été réalisées à l'aide de 3 types d'implants conformément à la norme ISO10993-6 (2007) :
- le Surgicel^{®}, pris comme référence ;
- l'implant Test1 correspond, quant à lui, à un matériau de type jersey, traité selon la première réaction d'oxydation (NaClO, TEMPO et NaClO₂) pendant une durée de 6 heures, puis selon la deuxième réaction d'oxydation au periodate pendant une durée de 6 heures ;
- l'implant Test2 a reçu un traitement équivalent à celui du Test1, à l'exception de la durée de la deuxième réaction d'oxydation au periodate, d'une durée de 3 heures seulement.

Ces études ont été menées sur 6 rats femelles de souche Sprague-Dawley. Les zones d'incision ont été tondues, les rats ont été anesthésiés. 2 incisions de chaque côté de la colonne vertébrale ont été effectuées (une incision dite craniale et une incision dite caudale). 4 implants de 1 x 1 cm ont été mis en place par animal. Les incisions du plan cutané ont été fermées par des agrafes.

Après 14 jours, les rats ont été sacrifiés par inhalation de CO₂. Les produits ont été retirés et les sites d'implantation ont été prélevés et analysés macroscopiquement.

Au site d'implantation a été évaluée la présence d'une nécrose, d'un exsudat, d'une néo vascularisation et d'une encapsulation, grâce à une grille de scores. L'échelle de note est la suivante : absent (0) ; réaction légère (1) ; réaction modérée (2) ; réaction marquée (3) ; réaction sévère (4).

Pour la résorption, c'est la persistance résiduelle du produit qui a été évaluée. L'échelle est la suivante :
- la note (0) est attribuée en cas de dégradation totale ;
- la note (1) est attribuée s'il reste des petits fragments du produit ;
- la note (2) est attribuée en cas de persistance modérée ;
- la note (3) est attribuée lorsque le produit est intact.

Les résultats sont présentés dans le Tableau 7 ci-dessous :

| | **Moyenne des scores (n= 3 rats, 4 sites d'implantation pour chaque produit*)** | | |
|---|---|---|---|
| **Condition** | **Test1 (periodate = 6h)** | **Test2 (periodate = 3h)** | **Référence (Surgicel®)** |
| **nécrose** | 0 | 0 | 0 |
| **exsudat** | 0 | 0 | 0,25 |
| **néovascularisation** | 0,5 | 0,5 | 0,5 |
| **encapsulation** | 0 | 0 | 0 |
| **résorption** | 0,25 | 1 | 0,67 |

| | | | |
|---|---|---|---|
| (*) seuls trois sites d'implantation ont été évalués pour le produit référence (Surgicel^{®}). | | | |

A J14, on observe une réaction légère à modérée des produits tests et de la compresse contrôle (exsudat et néo vascularisation). Les produits tests sont majoritairement résorbés ; pour le produit Test1, 3 sites sont complètement résorbés sur 4 et 1 site montre des petits fragments ; pour le produit Test2, 3 sites sont non résorbés ; enfin, pour le produit contrôle, 2 sites sur trois sont résorbés et 1 site montre une persistance modérée. Ainsi, le matériau obtenu par le procédé, objet de l'invention, possède une vitesse de résorption comparable au produit commercial référence Surgicel^{®}, et même meilleure, si l'on considère que le tissu utilisé dans cette étude a un grammage supérieur à celui du Surgicel^{®}.

Par ailleurs, ces études mettent en évidence que plus la durée de la réaction d'oxydation au periodate est longue, plus la résorption est rapide. Ainsi, le contrôle de la durée de la deuxième étape du procédé selon l'invention permet de moduler la vitesse de résorption du matériau cellulosique, en fonction de l'application qui lui est destinée.

### 2) Hémostase :

La performance hémostatique a été testée sur le textile cellulosique traité chez l'animal sur un modèle de saignement *per* opératoire versus le produit de référence Surgicel®. La méthodologie consiste à créer chez le porc des lésions de forme carrée et de surface prédéterminée sur des organes (rate et foie) puis de recouvrir les plaies ainsi créées des compresses à tester.

Les observations portent sur le comportement au contact du sang et le temps nécessaire pour établir l'hémostase (cf Tableau 8 ci-dessous). Foie et rate ont été choisis comme organe test car à la fois leur nature tissulaire et l'intensité du saignement qu'ils engendrent sont différentes.

| Organe | Temps d'hémostase | |
|---|---|---|
| | Textile cellulosique traité | Surgicel® |
| rate | 9 minutes | 10 minutes |
| foie | 2min 45' | 3 min |

On observe un comportement hémostatique du textile cellulosique testé comparable à celui du produit de référence, quel que soit l'organe testé: le comportement au contact du sang est identique à celui de la référence et le temps nécessaire pour obtenir l'arrêt du saignement équivalent pour chaque organe considéré.

Cette étude permet de mettre en évidence le pouvoir hémostatique du matériau.

### IV/ Caractéristiques du produit obtenu par inversion des étapes 1 et 2 :

Les conditions générales sont rassemblées dans le Tableau 9 ci-après :

| | |
|---|---|
| NaIO₄ (mole par gramme de cellulose) | 0,006 |
| pH du milieu réactionnel | 3,0 |
| Température (°C) | 35°C |
| Durée de la réaction | 3h |

### Etape 1 :

10g de cellulose sont mélangés avec 0,06mole d'ion périodate dans un erlen de 1L. Le volume total du mélange réactionnel est de 505mL. Le pH est ajusté à 3,0 et la réaction est réalisée dans un bain-marie sous agitation discontinue pendant 3h à une température de 35°C et à l'abri de la lumière. Le matériau cellulosique obtenu est ensuite rincé deux fois à l'eau purifiée avant de subir une acidification puis lavé plusieurs fois à l'éthanol. Après essorage le matériau est séché pendant 16h sous flux d'air.

### Etape 2 :

Le matériau précédent, obtenu à l'état sec, est soumis à la réaction TEMPO/NaClO/NaClO2. Les conditions générales sont rassemblées dans le Tableau 10 ci-après :

| | |
|---|---|
| TEMPO (mole par gramme de cellulose) | 0,0006 |
| NaClO (mole par gramme de cellulose) | 0,0012 |
| NaClO₂ (mole par gramme de cellulose) | 0,012 |
| pH du milieu réactionnel | 5,8 |
| Température (°C) | 60 |
| Durée de la réaction | 5h |

Sous hotte aspirante, 200mL d'une solution tampon d'acétate de sodium (0,5M, pH 5,8) sont placés dans un erlen de 1L puis 0,12mole de NaClO2 sont ajoutés. 10,1g du matériau obtenu précédemment sont ensuite placés dans la solution de NaClO2. 0,006mole de TEMPO et une solution aqueuse de NaClO à 2% en chlore actif (0,012mol) sont ensuite ajoutés au mélange réactionnel. Le volume réactionnel est ajusté à 500mL avec de la solution tampon acétate de sodium. L'erlen est fermé et placé dans un bain-marie thermostaté à la température de 60°C pendant 5h.

A l'issue de la réaction, le textile a disparu du milieu réactionnel. Le milieu réactionnel est alors précipité en présence d'éthanol mais le précipité obtenu est inexploitable.

### REFERENCES

Fujisawa S, Okita Y, Fukuzumi H, Saito T, Isogai A. 2011. Preparation and characterization of TEMPO-oxidized cellulose nanofibril films with free carboxyl groups. Carbohydrate Polymers 84(1):579-583.
Kumar V, Yang T. 1999. Analysis of carboxyl content in oxidized celluloses by solid-state 13C CP/MAS NMR spectroscopy. Int J Pharm. Jul 20;184(2):219-26.
Singh M, Ray AR, Vasudevan P, Verma K, Guha SK. 1979. Potential biosoluble carriers: biocompatibility and biodegradability of oxidized cellulose. Biomater Med Devices Artif Organs, 7(4):495-512.
Sobue H, Okubo M. 1956. Determination of carboxyl group in cellulosic materials with the "dynamic ion ex change method". Tappi, 39(6) :415.

## Revendications

1. Procédé d'obtention d'un matériau solide à base d'un polymère dont les unités cellobiose présentent les caractéristiques suivantes :
◆ au moins une partie des unités cellobiose présente au moins une fonction acide carboxylique porté par le carbone C₆, les autres carbones C₆ portant une fonction alcool primaire ; et
◆ au moins une partie des unités cellobiose présente au moins un des deux cycles ouvert entre les carbones C₂ et C₃, les autres carbones C₂ et C₃ formant un cycle et portan une fonction alcool
comprenant les étapes suivantes :
- une première étape de mise en contact d'un matériau solide à base de polymère contenant des unités cellobiose et d'un mélange oxydant comprenant un hypohalite, un halite et un sel d'oxoammonium ou un précurseur dudit sel ; puis
- une deuxième étape de mise en contact du matériau ainsi traité et d'une solution d'acide periodique ou de l'un de ses sels.

2. Procédé d'obtention d'un matériau selon la revendication 1, ***caractérisé* en ce qu'**il comprend une troisième étape de mise en contact du matériau ayant subi les deux premières étapes et d'une solution d'halite.

3. Procédé d'obtention d'un matériau selon la revendication 1 ou 2, ***caractérisé* en ce que** le mélange oxydant comprend de 0,0003 à 0,0006 mole par gramme de polymère de sel d'oxoammonium ou d'un précurseur dudit sel, avantageusement du TEMPO.

4. Procédé d'obtention d'un matériau selon l'une des revendications 1 à 3, ***caractérisé* en ce que** le mélange oxydant comprend de 0,0006 à 0,0049 mole par gramme de polymère d'hypohalite, avantageusement de l'hypochlorite de sodium.

5. Procédé d'obtention d'un matériau selon l'une des revendications 1 à 4, ***caractérisé* en ce que** le mélange oxydant comprend de 0,006 à 0,025 mole par gramme de polymère d'halite, avantageusement du chlorite de sodium.

6. Procédé d'obtention d'un matériau selon l'une des revendications 1 à 5, ***caractérisé* en ce que** la solution d'acide periodique ou de l'un de ses sels comprend de 0,003 à 0,012 mole par gramme de polymère, avantageusement 0,006 mole par gramme de polymère, d'acide periodique ou de l'un de ses sels, préférentiellement du periodate de sodium.

7. Procédé d'obtention d'un matériau selon l'une des revendications 2 à 6, ***caractérisé* en ce que** la solution d'halite comprend de 0,0025 à 0,012 mole par gramme de polymère, avantageusement 0,006 mole par gramme de polymère, d'halite, préférentiellement du chlorite de sodium.

8. Procédé d'obtention d'un matériau selon l'une des revendications 1 à 7, ***caractérisé* en ce que**, entre les deux premières étapes et/ou après la deuxième étape et/ou après la troisième étape, le matériau est incubé dans un milieu de protonation, avantageusement dans un ou plusieurs bains d'acide chlorhydrique (HCl).

9. Procédé d'obtention d'un matériau selon la revendication 8, ***caractérisé* en ce qu'**après l'étape de protonation, le matériau est soumis à une étape de lavage et éventuellement de séchage.

10. Matériau solide susceptible d'être obtenu à l'aide du procédé selon l'une des revendications 1 à 9 ***caractérisé* en ce que** :
- le matériau est un textile ;
- le matériau est à base d'un polymère dont les unités cellobiose présentent les caractéristiques suivantes :
◆ au moins une partie des unités cellobiose présente au moins une fonction acide carboxylique porté par le carbone C₆, les autres carbones C₆ portant une fonction alcool primaire ; et
◆ au moins une partie des unités cellobiose présente au moins un des deux cycles ouvert entre les carbones C₂ et C₃, les autres carbones C₂ et C₃ formant un cycle et portant une fonction alcool.

11. Matériau solide selon la revendication 10, ***caractérisé* en ce que** le polymère est de la cellulose ou de la viscose.

12. Matériau solide selon la revendication 10 ou 11, ***caractérisé* en ce que** les carbones C₂ et C₃ portent une fonction aldéhyde, éventuellement fonctionnalisée.

13. Matériau solide selon l'une des revendications 10 à 12, ***caractérisé* en ce que** les carbones C₂ et/ou C₃ portent une fonction acide carboxylique, éventuellement fonctionnalisée.

14. Matériau solide selon l'une des revendications 10 à 13, ***caractérisé* en ce qu'**il présente un taux d'oxydation global supérieur à 10%, avantageusement supérieur à 12%.

15. Matériau solide selon l'une des revendications 10 à 14 pour son utilisation comme compresse.

## Patentansprüche

1. Verfahren zur Herstellung eines festen Materials auf Grundlage eines Polymers, dessen Cellobiose-Einheiten die folgenden Eigenschaften aufweisen:
• zumindest ein Teil der Cellobiose-Einheiten weist mindestens eine Carbonsäurefunktion auf, getragen vom C6- Kohlenstoffatom, die anderen C6-Kohlenstoffatome tragen eine primäre Alkoholfunktion; und
• zumindest ein Teil der Cellobiose-Einheiten weist mindestens einen der beiden Ringe auf, der zwischen den C2 und C3 Kohlenstoffatomen offen ist, die anderen C2 und C3 Kohlenstoffatomen bilden einen Ring und tragen eine Alkoholfunktion
das die folgenden Schritte beinhaltet:
- ein erster Schritt, in dem ein festes Material, auf Polymer- Grundlage, das Cellobiose- Einheiten enthält, in Kontakt gebracht wird mit einer oxydierenden Mischung, die ein Hypohalit, ein Halit und ein Oxoammoniumsalz, oder einen Vorläufer dieses Salzes enthält; dann
- einen zweiten Schritt, in dem das so behandelte Material mit einer Lösung aus Perjodsäure oder einem ihrer Salze in Kontakt gebracht wird.

2. Verfahren zur Herstellung eines Materials gemäß Anspruch 1, ***dadurch gekennzeichnet,* dass** es einen dritten Schritt umfasst, in dem das Material, das die ersten beiden Schritte durchlaufen hat, mit einer Halit- Lösung in Kontakt gebracht wird.

3. Verfahren zur Herstellung eines Materials gemäß Anspruch 1 oder 2, ***dadurch gekennzeichnet,* dass** die oxydierende Mischung zwischen 0,0003 und 0,0006 Mol pro Gramm Polymer Oxoammoniumsalz, oder eines Vorläufer dieses Salzes enthält, vorzugsweise TEMPO.

4. Verfahren zur Herstellung eines Materials gemäß einem der Ansprüche 1 bis 3, ***dadurch gekennzeichnet,* dass** die oxydierende Mischung zwischen 0,0006 und 0,0049 Mol pro Gramm Polymer Hypohalit enthält, vorzugsweise Natriumhypochlorit.

5. Verfahren zur Herstellung eines Materials gemäß einem der Ansprüche 1 bis 4, ***dadurch gekennzeichnet*, dass** die oxydierende Mischung zwischen 0,006 und 0,025 Mol pro Gramm Polymer Halit enthält, vorzugsweise Natriumchlorit.

6. Verfahren zur Herstellung eines Materials gemäß einem der Ansprüche 1 bis 5, ***dadurch gekennzeichnet*, dass** die Lösung aus Perjodsäure oder einem ihrer Salze zwischen 0,003 und 0,012 Mol pro Gramm Polymer enthält, vorzugsweise 0,006 Mol pro Gramm Polymer, Perjodsäure oder eines ihrer Salze, am besten Natriumperjodat.

7. Verfahren zur Herstellung eines Materials gemäß einem der Ansprüche 2 bis 6, ***dadurch gekennzeichnet,* dass** die Halit- Lösung zwischen 0,0025 und 0,012 Mol pro Gramm Polymer, besser noch 0,006 Mol pro Gramm Polymer, Halit enthält, vorzugsweise Natriumchlorit.

8. Verfahren zur Herstellung eines Materials gemäß einem der Ansprüche 1 bis 7, ***dadurch gekennzeichnet*, dass** zwischen den beiden ersten Schritten und/oder nach dem zweiten Schritt und/oder nach dem dritten Schritt, das Material in einem Protonierungmilieu inkubiert wird, vorteilhafterweise in einem oder mehreren Salzsäure- (HCl)- Bädern.

9. Verfahren zur Herstellung eines Materials gemäß Anspruch 8, ***dadurch gekennzeichnet,* dass** nach dem Schritt der Protonierung des Materials ein Schritt des Waschens und eventuell Trocknens folgt.

10. Festes Material, erhalten von dem Verfahren gemäß einem der Ansprüche 1 bis *9,* ***dadurch gekennzeichnet*, dass**
- es sich bei dem Material um einen Stoff handelt;
- das Material auf der Grundlage eines Polymers beruht, dessen Cellobiose-Einheiten die folgenden Eigenschaften aufweisen:
• zumindest ein Teil der Cellobiose-Einheiten weist mindestens eine Carbonsäurefunktion auf, die vom C6- Kohlenstoffatom getragen wird, die anderen C6- Kohlenstoffatome tragen eine primäre Alkoholfunktion; und
• zumindest ein Teil der Cellobiose-Einheiten weist mindestens einen der beiden Ringe auf, der zwischen den C2 und C3 Kohlenstoffatomen offen ist, die anderen C2 und C3 Kohlenstoffatomen bilden einen Ring und tragen eine Alkoholfunktion.

11. Festes Material gemäß Anspruch 10, ***dadurch gekennzeichnet,* dass** es sich bei dem Polymer um Cellulose oder Viskose handelt.

12. Festes Material gemäß Anspruch 10 oder 11, ***dadurch gekennzeichnet*, dass** die Kohlenstoffatome C₂ und/oder C₃ eine Aldehyd- Funktion tragen, eventuell funktionalisiert.

13. Festes Material gemäß einem der Ansprüche 10 bis 12, ***dadurch gekennzeichnet*, dass** die Kohlenstoffatome C₂ und C₃ eine Carbonsäure - Funktion tragen, eventuell funktionalisiert.

14. Festes Material gemäß einem der Ansprüche 10 bis 13, ***dadurch gekennzeichnet,* dass** er einen Gesamtoxidationsgrad von über 10 %, besser noch von über 12 % aufweist.

15. Festes Material gemäß einem der Ansprüche 10 bis 14, zur Verwendung als Kompresse.

## Claims

1. A method of obtaining a solid material based on a polymer having its cellobiose units exhibiting the following characteristics:
◆ at least some of the cellobiose units have at least one carboxylic acid function attached to the C₆ carbon, the other C₆ carbons having a primary alcohol function attached thereto; and
◆ at least some of the cellobiose units have at least one of the two rings open between the C₂ and C₃ carbons, the other C₂ and C₃ carbons forming a ring and having an alcohol function attached thereto
comprising the following steps:
- a first step of placing in contact a solid material based on polymer containing cellobiose units and an oxidizing mixture comprising a hypohalite, a halite, and an oxoammonium salt or a precursor of said salt; and then
- a second step of placing in contact the material thus processed and a solution of periodic acid or of a salt thereof.

2. The method of obtaining a material of claim 1, ***characterized in that*** it comprises a third step of placing in contact the material having been submitted to the first two steps and a halite solution.

3. The method of obtaining a material of claim 1 or 2, ***characterized in that*** the oxidizing mixture comprises from 0.0003 to 0.0006 mole per gram of polymer of oxoammonium salt or a precursor of said salt, advantageously TEMPO.

4. The method of obtaining a material of any of claims 1 to 3, ***characterized in that*** the oxidizing mixture comprises from 0.0006 to 0.0049 mole per gram of polymer of hypohalite, advantageously sodium hypochlorite.

5. The method of obtaining a material of any of claims 1 to 4, ***characterized in that*** the oxidizing mixture comprises from 0.006 to 0.025 mole per gram of polymer of halite, advantageously sodium chlorite.

6. The method of obtaining a material of any of claims 1 to 5, ***characterized in that*** the solution of periodic acid or of a salt thereof comprises from 0.003 to 0.012 mole per gram of polymer, advantageously 0.006 mole per gram of polymer, of periodic acid or of a salt thereof, preferably sodium periodate.

7. The method of obtaining a material of any of claims 2 to 6, ***characterized in that*** the halite solution comprises from 0.0025 to 0.012 mole per gram of polymer, advantageously 0.006 mole per gram of polymer, of halite, preferably sodium chlorite.

8. The method of obtaining a material of any of claims 1 to 7, ***characterized in that*** between the first two steps and/or after the second step and/or after the third step, the material is incubated in a protonation medium, advantageously in one or a plurality of hydrochloric acid (HCl) baths.

9. The method of obtaining a material of claim 8, ***characterized in that*** after the protonation step, the material is submitted to a step of washing and possibly drying.

10. A solid material capable of being obtained by means of the method of any of claims 1 to *9, **characterized in that***:
- the material is a textile;
- the material is based on a polymer having its cellobiose units exhibiting the following characteristics:
◆ at least some of the cellobiose units have at least one carboxylic acid function attached to the C₆ carbon, the other C₆ carbons having a primary alcohol function attached thereto; and
◆ at least some of the cellobiose units have at least one of the two rings open between the C₂ and C₃ carbons, the other C₂ and C₃ carbons forming a ring and having an alcohol function attached thereto.

11. The solid material of claim 10, ***characterized in that*** the polymer is cellulose or viscose.

12. The solid material of claim 10 or 11, ***characterized in that*** the C₂ and C₃ carbons have an aldehyde function, possibly functionalized, attached thereto.

13. The solid material of any of claims 10 to 12, ***characterized in that*** the C₂ and/or C₃ carbons have a carboxylic acid function, possibly functionalized, attached thereto.

14. The solid material of any of claims 10 to 13, ***characterized in that*** it has a global oxidation level greater than 10%, advantageously greater than 12%.

15. The solid material of any of claims 10 to 14 for use as a compress.
